# EUROPÄISCHE PATENTSCHRIFT

(11) **EP 2 349 114 B1**
(45) Veröffentlichungstag und Bekanntmachung des Hinweises auf die Patenterteilung: **21.10.2015**
(21) Anmeldenummer: 09772466.0
(22) Anmeldetag: 01.07.2009
(51) Int. Cl.: A61L 27/10, C04B 35/119, C04B 35/42, A61F 2/30, A61F 2/44

(54) **BANDSCHEIBENENDOPROTHESE**
INTERVERTEBRAL DISC ENDOPROSTHESIS
ENDOPROTHÈSE DE DISQUE INTERVERTÉBRAL

(30) Priorität: 03.07.2008 DE 102008040115
(43) Veröffentlichungstag der Anmeldung: 03.08.2011
(73) Patentinhaber: CeramTec GmbH, 73207 Plochingen (DE); Synthes GmbH, 4436 Oberdorf (CH)
(72) Erfinder: NIESS, Christine, 72669 Unterensingen (DE); PREUSS, Roman, 73230 Kirchheim unter Teck (DE); SCHNEIDER, Norbert, 73614 Schorndorf (DE); GRÄSSEL, Matthias, 73760 Ostfildern (DE)
(74) Vertreter: Scherzberg, Andreas Hans
(86) Internationale Anmeldenummer: PCT/EP2009/058238
(87) Internationale Veröffentlichungsnummer: WO 2010/000766

(56) Entgegenhaltungen:
- WO-A-2006/119088
- WO-A1-92/02470
- DE-A1- 10 323 363
- DE-A1- 19 850 366
- US-A1- 2007 135 923

## Beschreibung

Die Erfindung betrifft eine Bandscheibenendoprothese nach dem Oberbegriff des Anspruchs 1.

Derzeit existierende Bandscheibenendoprothesen (Total Disc Replacements) bestehen aus zwei metallischen Endplatten, die ein Einwachsen des angrenzenden Wirbelkörper-Knochens durch Ihre makroskopische und mikroskopische Oberflächengestalt und ggf. zusätzlichen bioaktiven Beschichtungen (z.B. Hydroxylapatit) ermöglichen sollen. In diese Endplatten sind beim so genannten "fixed ball and socket" Prinzip jeweils eine hauben- und eine kappenförmige Gleitoberfläche aus Metall, Kunststoff oder Keramik integriert, welche durch ihr aufeinander gleiten die Beweglichkeit des operierten Segments erhält bzw. rekonstruiert. Alle metallischen Endplatten haben den Nachteil mehr oder weniger starke, unerwünschte Artefakte bei der Magnet Resonanz Tomographie (MRT) zu erzeugen, welche die Qualität der erzeugten Bilder verschlechtert.

US 2007/0135923 A1 zeigt eine Bandscheibenendoprothese zur Implantation in die Wirbelsäule, mit einer ober- und unterseitigen Endplatte zur jeweiligen Befestigung an den Wirbelkörper-Knochen und einem zwischen den Endplatten angeordneten Gleitlager, bestehend aus Gleitkörpern, die aufeinander gleitend ausgebildet sind, wobei beide Endplatten aus Kunststoff und die Gleitkörper aus Keramik bestehen, die Gleitkörper vom Kunststoff der Endplatten umspritzt sind und die vom Kunststoff der Endplatten umspritzten Oberflächenbereiche der Gleitkörper im Vergleich zu ebenen Flächen Oberflächenvergrößerungen aufweisen.

Der Erfindung liegt die Aufgabe zugrunde, eine Bandscheibenendoprothese nach dem Oberbegriff des Anspruchs 1 so zu verbessern, dass die Gleitkörper dauerhaft fest und verdrehsicher mit den Endplatten verbunden sind.

Erfindungsgemäß wird diese Aufgabe dadurch gelöst, dass die Oberflächenvergrößerungen Freiräume in den Gleitkörpern sind.

In diese Freiräume dringt der Kunststoff der Endplatten ein und stellt dadurch eine feste und verdrehsichere Verbindung her.

Die Freiräume sind mindestens eine auf dem Außenumfang der Gleitkörper umlaufende Nut. Umlaufende Nute sind leicht herzustellen und führen zu einer festen und verdrehsicheren Verbindung. Diese Nuten sind entweder vor oder nach dem Sintern in die Gleitkörper einzubringen. Bevorzugt werden sie vor dem Sintern in den Grünkörper eingebracht.

Die erfindungsgemäße Bandscheibenendoprothese wird an einer Halswirbelsäule oder Lendenwirbelsäule verwendet.

Dadurch, dass bevorzugt knochenseitig beschichtete Kunststoffendplatten, z.B. aus PEEK oder PEKK zum Einsatz kommen, sind MRT Artefakte vermieden und gleichzeitig ein Knocheneinwachstum ermöglicht. Diese per Spritzguss hergestellten Kunststoff-Endplatten integrieren die Gleitkörper aus Keramik. Um diese am Herausfallen zu hindern und um eine Rotationssicherung zu gewährleisten, sind eine aufgeraute Oberfläche (Mikroformschluss), Freiräume oder andersartige Geometrieelemente in den vergossenen Teil der Gleitpartner notwendig, die mit dem Kunststoff beim Spritzgießen ausgefüllt bzw. umschlossen werden, z.B. in Form einer umlaufenden Nut, Bohrungen oder Erhebungen, wie z.B. Spikes.

Weitere Merkmale der Erfindung ergeben sich aus den Figuren, die nachfolgend näher beschrieben sind.

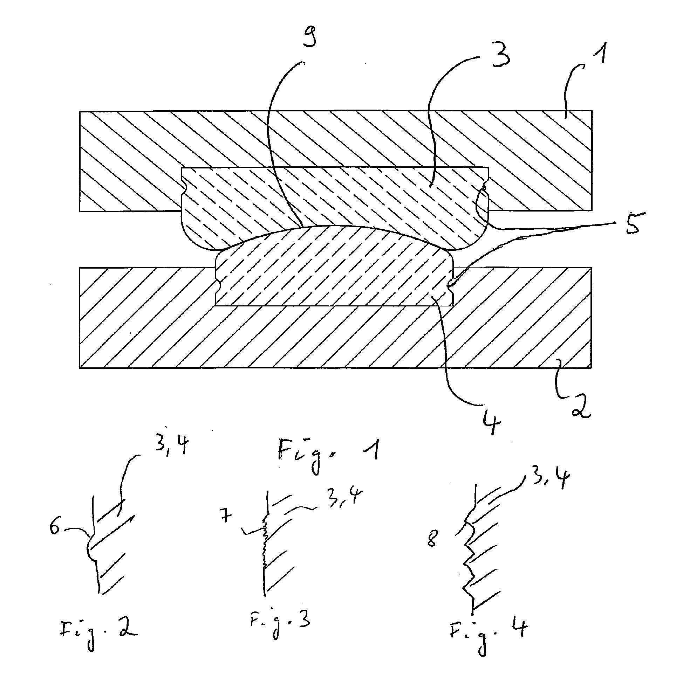

Figur 1 zeigt eine Bandscheibenendoprothese zur Implantation in die Halswirbelsäule. Die Bandscheibenendoprothese besteht aus einer oberseitigen 1 und einer unterseitigen Endplatte 2 aus Kunststoff, z. B. PEEK oder PEKK.

In diese Endplatten 1, 2 ist jeweils ein Gleitkörper 3, 4 eines Gleitlagers eingebettet. Dies ist dadurch erreicht worden, dass die Gleitkörper 3, 4 vom Kunststoff der Endplatten 1, 2 umspritzt sind. Die Gleitfunktion des Gleitlagers ist durch zwei aufeinander gleitenden Flächen 9 erreicht. Das Gleitlager kann auch aus mehr als zwei Gleitkörpern bestehen.

Zur drehsicheren Verankerung weisen die vom Kunststoff der Endplatten 1, 2 umspritzten Oberflächenbereiche der Gleitkörper 3, 4 im Vergleich zu ebenen Flächen Oberflächenvergrößerungen auf.

In Figur 1 ist die Oberflächenvergrößerung eine auf dem Außenumfang der Gleitkörper 3, 4 umlaufende Nut 5. In diese Nut 5 dringt der Kunststoff der Endplatten 1, 2 ein.

Figur 2 zeigt in einem Ausschnitt eine Erhebung 6 auf dem Außenumfang der Gleitkörper 3, 4.

Figur 3 zeigt in einem Ausschnitt eine Aufrauung 7 auf dem Außenumfang der Gleitkörper 3, 4.

Figur 4 zeigt in einem Ausschnitt Spikes 8 auf dem Außenumfang der Gleitkörper 3, 4.

Die Bandscheibenendoprothese wird an einer Halswirbelsäule oder Lendenwirbelsäule verwendet.

## Patentansprüche

1. Bandscheibenendoprothese mit einer ober- (1) und unterseitigen Endplatte (2) und einem zwischen den Endplatten (1, 2) angeordneten Gleitlager, bestehend aus Gleitkörpern (3, 4), die aufeinander gleitend ausgebildet sind, wobei beide Endplatten (1, 2) aus Kunststoff und die Gleitkörper (3, 4) aus Keramik bestehen, die Gleitkörper (3, 4) vom Kunststoff der Endplatten (1, 2) umspritzt sind und die vom Kunststoff der Endplatten (1, 2) umspritzten Oberflächenbereiche der Gleitkörper (3, 4) im Vergleich zu ebenen Flächen Oberflächenvergrößerungen aufweisen, wobei die Oberflächenvergrößerungen Freiräume in den Gleitkörpern (3, 4) sind und die Freiräume mindestens eine auf dem Außenumfang der Gleitkörper (3, 4) umlaufende Nut (5) sind zur Verwendung an einer Halswirbelsäule oder Lendenwirbelsäule.

## Claims

1. Intervertebral disc endoprosthesis, having an upper end plate (1) and a lower end plate (2) and a sliding bearing which is arranged between the end plates (1, 2) and is composed of sliding bodies (3, 4) that are designed to slide on each other, wherein both end plates (1, 2) are made of plastic and the sliding bodies (3, 4) are made of ceramic, the sliding bodies (3, 4) are encapsulated by the plastic of the end plates (1, 2), and the surface regions of the sliding bodies (3, 4) encapsulated by the plastic of the end plates (1, 2) have surface-area enlargements by comparison with flat surfaces, wherein the surface-area enlargements are clearances in the sliding bodies (3, 4), and the clearances are at least one circumferential groove (5) on the outer circumference of the sliding bodies (3, 4), for use on a cervical spinal column or lumbar spinal column.

## Revendications

1. Endoprothèse de disque intervertébral, comprenant une plaque d'extrémité supérieure (1) et une plaque d'extrémité inférieure (2) et un palier lisse disposé entre les plaques d'extrémité (1, 2) et constitué de corps de glissement (3, 4) qui sont réalisés de manière à glisser l'un sur l'autre, les deux plaques d'extrémité (1,2) étant constituées de matière plastique, et les corps de glissement (3, 4) étant constitués de céramique, les corps de glissement (3, 4) étant enrobés par moulage par injection avec la matière plastique des plaques d'extrémité (1, 2), et les zones de surface des corps de glissement (3, 4), qui sont enrobées par moulage par injection avec la matière plastique des plaques d'extrémité (1, 2), présentant des augmentations de surface comparées à des surfaces planes, les augmentations de surface étant des espaces libres dans les corps de glissement (3, 4), et les espaces libres étant au moins une gorge (5) s'étendant sur la périphérie extérieure des corps de glissement (3, 4), en vue de l'utilisation sur une colonne de vertèbres cervicales ou une colonne de vertèbres lombaires.
